# EUROPEAN PATENT SPECIFICATION

(11) **EP 3 340 952 B1**
(45) Date of publication and mention of the grant of the patent: **07.06.2023**
(21) Application number: 16839704.0
(22) Date of filing: 24.08.2016
(51) Int. Cl.: A61F 13/08, A61F 5/30, A61F 13/06

(54) **A KIT AND THERAPEUTIC PRESSURE ASSEMBLY WITH PATCHES FOR APPLYING PRESSURE TO A LIMB OR OTHER BODY PART**
KIT UND THERAPEUTISCHE DRUCKANORDNUNG MIT PFLASTERN ZUM AUSÜBEN VON DRUCK AUF EIN GLIED ODER EINEN ANDEREN KÖRPERTEIL
KIT ET ENSEMBLE DE PRESSION THÉRAPEUTIQUE À PATCHS PERMETTANT D'APPLIQUER UNE PRESSION SUR UN MEMBRE OU UNE AUTRE PARTIE DU CORPS

(30) Priority: 27.08.2015 SE 1551117
(43) Date of publication of application: 04.07.2018
(73) Proprietor: Presscise AB, 524 30 Herrljunga (SE)
(72) Inventor: LUNDH, Torbjörn, 427 35 Billdal (SE); DAMM, Josefin, 524 95 Ljung (SE); VASILIS, Jonatan, 411 29 Göteborg (SE)
(74) Representative: Lind Edlund Kenamets Intellectual Property AB
(86) International application number: PCT/SE2016/050792
(87) International publication number: WO 2017/034462

(56) References cited:
- WO-A1-2015/007335
- WO-A2-00/15139
- GB-A- 2 474 440
- US-A1- 2007 179 421
- US-A1- 2010 024 100
- US-A1- 2012 179 084
- US-A1- 2015 157 524

## Description

### Technical field of the invention

The present invention relates to a therapeutic pressure assembly and a kit for making a limb or other body part encircling therapeutic device, for applying dynamic and/or static pressure to said limb/body part.

### Background

Compression assemblies or devices, and in particular compression garments such as compression socks, compression bandages, tubular bandages, pantyhoses, girdles and sleeves, are being used to obtain a static additional sub-bandage pressure for several indications, such as venous insufficiency, oedema treatment, after surgery or treatment of varicose veins, relief in case of injuries such as subcutaneous bleeding associated with blunt trauma, muscle sprains, to support blood circulation for persons that might have poor circulation, have to stand for a long time, to perform (or recover from) sport activities, or other physically demanding activities.

It is also well known that flexing the calf - for example when walking - will result in a pumping effect that will enhance the venous return flow and hence decrease the degree, or risk, of indications such as leg ulcers and oedema. This pumping effect may be referred to as a dynamic pressure. In order to improve this pumping effect, one might apply a stiff, or low stretch, bandage on the calf to dynamically increase the pressure peaks resulting in a massaging effect that will further improve the proximal venous return flow.

In sports, elastic compression garments are today being used in order to: improve recovery, faster warm up and enhance overall circulation, enhance stability and agility, reduce fatigue and muscle damage and treat injuries such as muscle strains or sprains, or minimize subcutaneous bleeding associated with blunt trauma.

All indications and problems discussed above are also applicable in different settings in veterinary medicine. The variety of indications needs a variety of different pressures to be applied for optimal function. Depending on indication, e.g. therapy or prevention, and targeted location, the needed pressure to be applied varies. However, providing an appropriate pressure, especially for compression therapy, is difficult in practice, and there is a great risk that compression is applied in a non-optimal way. Since the needed pressure varies with the indication and condition, the optimal pressure distribution is difficult to reach. For example, if an athlete gets a big contusion, one would like to localize a well-defined pressure profile on the center of that contusion.

The pressure exerted on a body part by a compression garment is determined by Laplace's law in terms of the shape of the body part and the hoop force in the compression garment. Typically, the body part has approximately circular cross sections, allowing the shape of the body part to be expressed in terms of the circumference, which is easy to measure. It follows from Laplace's law that, on the one hand, the pressure increases with increasing hoop stress and, on the other hand, decreases with increasing circumference.

However, increasing the size of a body part - which in itself would cause the pressure to decrease - must also cause the compression garment to become more stretched, which typically increases the hoop stress. That is, whether the resulting pressure increases, decreases or even stays constant depends on the properties of the material in the compression garment.

Further, when a patient is walking, the circumference of the legs will naturally vary due to the flexing of the calves muscles. Furthermore, just standing up will cause an increase in circumference due to gravitational effects.

It is sometimes a desirable feature that a small dynamic increase in circumference of a body part wearing a compression garment should yield a significant dynamic increase in pressure. That is, the hoop stress should rapidly increase with increasing elongation. However, this implies that the stretching of the compression garment must be controlled with high precision. If the initial stretching of the garment is not enough for the specific circumference, then the static pressure can be much too low. If the garment is instead stretched just a little too much, then the static pressure will be much higher than desired.

One approach towards achieving both a dynamic increase in pressure and a controlled static pressure, disclosed in US6338723 and US7329232, is to use a garment with a plurality of straps which are secured at a specific elongation, determined by an estimated size of the body part. A set of scales for different body part sizes can be provided on the compression garment or on a reference card, separate from the compression garment. However, the same garment and material is responsible for both the dynamic increase in pressure and the static pressure, and very precise measurements are required to obtain adequate static and dynamic pressure levels. Furthermore, to simplify the scale, it is necessary to quantify the size of the body part in discrete groups (such as small, medium and large), so that the pressure is only known within a certain pressure range, making the garment less controllable.

Another approach uses multiple components, where different components offer the primary contribution to the dynamic increase in pressure and the static pressure, respectively. For instance, US20120238933 discloses a two-layer compression bandage system, where the inner component can be characterized as "short stretch", which will create a dynamic increase in pressure, and the outer layer primarily creates the static pressure.

Similarly, WO2015007335, by the same applicant, discloses an elastic bandage that exerts a constant static pressure, even when the size of the body part increases, and which can be combined with a second bandage with "short stretch", applied at close to zero stretch and optionally fastened in the elastic bandage.

In practice, however, it is very difficult to properly apply the "short stretch" bandage layer. It is desirable that the bandage material is applied in a stretched state, as this makes it easier for the bandage to stay attached to and adapt to the shape of the body part. However, stretching it just a little too much will cause the second bandage layer to contribute to or even dominate the static pressure, and if it is not stretched enough, then the dynamic increase in pressure might disappear.

Also, use of a completely inelastic bandage material for the second bandage layer and application of the layer essentially unstretched would not be a remedy to this problem. First of all, this would make it more difficult for the bandage to stay attached to and adapt to the shape of the body part. Further, this results in essentially the same problems as in the first case. A zero stretching force when applying the bandage tends to cause folds in the bandage, folds which will expand when the size of the body part increases, eliminating the desired dynamic increase in pressure. On the other hand, applying the bandage with "almost" zero stretch is very difficult even for experienced bandage appliers, and will again often result in the second bandage layer contributing to or even dominating the static pressure.

Thus, there is a need for a compression assembly or device that provides a more efficient and controllable pressure on the limb or other body part when applied, which is easier to apply on the user's limb/body part, and/or which is more cost-efficient to produce. In particular, there is a need for a compression assembly or device which yields a dynamic increase in pressure on a body part when its size changes, that exerts zero or otherwise controlled static pressure, that adapts to body parts of varying size and shape, and/or that does not require measurements when applying the compression garment.

### Summary of the invention

There is therefore an object of the present invention to provide an assembly and a kit which at least partly alleviates the above-discussed drawbacks, and at least partly addresses the above-identified needs.

This object is achieved by means of an assembly and a kit in accordance with the appended claims.

According to a first aspect of the present invention, there is provided a kit for making a limb or other body part encircling therapeutic device, for applying dynamic and/or static pressure to said limb/body part, in accordance with claim 1. -

According to still another aspect of the invention, there is provided a therapeutic pressure assembly for encircling a limb or other body part and for applying dynamic and/or static pressure to said limb/body part, in accordance with claim 11.

The present invention is based on the understanding that in order for pressure therapy to work efficiently, there is a need for a device or assembly which is versatile and adaptable, easy to use, and which provides controllable and predictable pressure on the limb or other body part to be treated. The present invention fulfills this. The underlying garment or bandage may be applied in an easy and conventional way, as is per se known, and may, if desired, be arranged to provide a static pressure on the limb/body part. This is then complemented by the layer of patches being arranged over the garment/bandage, to provide a controllable dynamic pressure. The patches are simple to attach and re-attach to the garment/bandage, and it is simple to attach the patches also for inexperienced users, users having reduced dexterity, being older or have other limitations of mobility or the like. The patches are placed in a slightly overlapping manner, thereby forming a patchwork overlying at least part of the garment/bandage. Forming the second layer with patches in this way makes it easy to encircle the limb/body part in any desired way, and on limbs/body parts having vastly different sizes, shapes, etc. Since the patches are arranged in an overlapping manner, and since the patches are not only attached to the underlying garment/bandage, but also, and preferably more strongly, to each other, a continuous second layer is formed by the patches, which is efficient to provide dynamic pressure to the limb/body part. It is also simple to address specific needs, and make local adaptations, such as providing low pressure areas, providing access openings, etc in the patch layer.

Arranging the second layer by patches in this way is also highly advantageous in that it can easily and reliably be arranged free or essentially free of static compression of the limb/body part. Since the patch layer is built up in situ over the limb/body part, and since it is built up by means of arranging patches in an overlapping fashion, no stress or stretch is applied to the patches, and consequently no static compression of the limb/body part occurs. Thus, the patch layer only contributes to the dynamic pressure, and the static pressure, if so desired, is provided solely by the garment/bandage. This separation makes it possible to control both the dynamic and the static pressure in a very efficient, predictable and simple way, and also to control these properties entirely independently of each other. Thus, the specific properties desired for any specific user and condition can therefore easily be tailored in a controllable way, and readjustment of any of the properties can also be made over time.

Thus, differently put the present invention is based on the use of double adhesive patches of a prescribed elasticity, size and shape in order to create a pressure component, with a certain stiffness, for use on e.g. the calves in order to create a massage effect to improve the venous flow back to the heart.

The set of patches are assembled in situ to form a compression assembly/device with a controlled static pressure and a dynamic increase in pressure when the size of the body part increases.

The garment/bandage may be of various per se known types, such as ordinary bandages, tube bandages, stockings and other type of compression garments. The garment or bandage may of the type providing a compression of the limb/body part. However, for some applications and therapeutic treatments, where only a dynamic pressure is required, and no static compression, the garment or bandage may be of a type exerting no static compression to the limb/body part. In particular, it is preferred to use a garment or bandage made of a material in accordance with WO2015007335, by the same applicant. This material has the advantage of exerting a very well defined and predictable static pressure on the limb/body part, which remains the same even when the size of the body part varies. Such an elastic bandage/garment preferably has the elastic property that when the same length of unstretched elastic material is stretched to encircle circular object with different circumferences at different yield rates the pressure exerted by the elastic material varies less than 30% over a range of approximately circular circumferences providing a range of yield rates from λ1 to λ2, wherein λ2/λ1 > 1.8. Preferably the pressure exerted by the material varies less than 20% over a range of circumferences providing a range of yield rates from λ1 to λ2, and preferably varies less than 10% Even more preferably, λ2/λ1 > 1.9, and most preferably λ2/λ1 > 2.0.

Use of such a garments/bandages provides particular advantages in combination with the above-discussed patch layer. Such garments/bandages provides an essentially constant static pressure, regardless of variations of shape and size in the limb/body part, and thus, exerts close to no dynamic pressure. At the same time, the patches may, as already discussed, provide essentially only a dynamic pressure - i.e. the pressure of the patch layer itself may be zero or close to zero when the limb/body part is in a rest position, and then temporarily and dynamically increase when the limb/body part is moved. Thus, the combination of such a garment/bandage and the patches allows the dynamic and static pressures to be controlled very precisely and independently. This makes it possible to apply the pressure profile that most efficiently treats the indication or indications at hand, as well as to make individual adjustments to increase patient comfort. This type of garment/bandage may also be configured or applied to provide close to static compression to the limb/body part. Hereby, only a dynamic pressure, provided by the patch layer, is applied to the limb/body part, which is advantageous for some types of therapeutic treatment.

The patches are preferably made of a material with short stretch, or non-elastic, and provided with at least one adhesive surface in order to strongly adhere to each other and adhere, at least weakly, to the underlying material. Each patch preferably has a shape and size that makes it difficult to accidentally stretch the material when applying the patch, and also makes it possible to adapt to body parts of different shapes.

The therapeutic assembly/device of the present invention is useable on different types of limbs, and "limb" should in the context of the present invention be construed broadly, indicating any extending part of the human or animal body, such as legs, arms and the head. The invention is particularly useful for pressure therapy on legs. However, the therapeutic assembly/device of the present invention is useable also on other body parts, such as on the trunk and the chest.

All patches have limited dimensions in all directions, compared to the circumferential dimension of said limb/body part. In particular, it is preferred that the patches are about the size of a human palm, or the like. For example, each patch may have a maximal extension being lower than 30 cm, and preferably less than 15 cm, and most preferably less than 10 cm. Further, the patches are dimensioned such that at least 3 patches are required to encircle the limb/body part, and preferably at least 5, and most preferably at least 7. Using patches of such sizes makes application simple, since the number of patches necessary to cover the desired area may hereby be sufficiently small to make mounting simple and quick, and yet sufficiently large to make it easy to form the patch layer in concurrence with the shape and size of the limb or other body part to be covered, and to avoid inadvertent stretching and compression by the patch layer.

The patches may have various shapes. In one embodiment, at least some of the patches have a tapered end, and preferably having a generally triangular or drop shape. In another embodiment, at least some of the patches have a bone shape, having greater dimensions towards opposite ends, and a narrower waist there between. The patch layer may be formed by use of a single type of patches, all having the same size and shape, or by use of two or more different types of patches, having e.g. different shapes and/or different sizes.

The patches are preferably arranged to attach more strongly to each other, at the overlap areas, and less strongly to the garment or bandage. This facilitates mounting, detachment and remounting of the patches, and at the same time makes the patch layer hold together more strongly.

Adherence and attachment of the patches may be provided in various ways. In a preferred embodiment, the patches comprise at least one of adhesive (glue) and a hook and loop fabric for attachment to other patches and/or to the garment or bandage.

The patches are preferably made of a substantially inelastic, non-stretchable material or low stretchable material. This makes the patch layer relatively inelastic and non-stretchable, which is advantageous for the desired dynamic pressure. However, it is also feasible to use patches which are stretchable.

The patches preferably comprise a marking around the edges indicating a designated overlap area. This facilitates mounting, and ensures that the overlap is always adequately dimensioned, and neither too large nor too small.

At least one of the patches may further be provided with one or more openings. Such opening(s) facilitates migration of air, vapour and liquid through the patch layer, and may also be used to create low pressure areas and the like.

The patches may be provided in a package, e.g. made of plastic film or the like, and be packed either separately or many together. If two or more patches are packed together, they may be arranged with the same type of adherent side facing each other, in order to be easier to separate from each other, or be separated by a separation layer, such as a plastic film layer, a sheet of paper or the like.

Preferably, a kit of the above-discussed type comprises a single package, containing a sufficient number of patches to cover body parts of normal size, and/or body parts for which the patches are specifically intended. The package may also comprise the bandage/garment, e.g. arranged in a separate compartment of the package. However, the bandage/garment may also be provided separately.

It is also possible to provide the patches in a semi-assembled way, where two or more patches are arranged connected to each other. This may facilitate application of the patch layer, and if necessary, some of the semi-attached patches may still easily be removed or re-arranged during application. Such patch groups of semi-attached patches are of particular advantage if many relatively small patches are provided.

These and other features and advantages of the present invention will in the following be further clarified with reference to the embodiments described hereinafter.

### Brief description of the drawings

For exemplifying purposes, the invention will be described in closer detail in the following with reference to embodiments thereof illustrated in the attached drawings, wherein:
Figs. 1a and 1b are perspective views of two patches illustrating the attachment of the patches to underlying garment or bandage and to other patches;
Figs. 2a and 2b are perspective views of the application of the patch assembly on a limb, and the patch assembly in an applied state, respectively, in accordance with an embodiment of the present invention;
Fig. 3 is a perspective view of a patch assembly similar to the one in Fig. 2, but arranged in a different way, thereby providing a different encircling of the body part;
Fig. 4a and 4b are perspective views of a patch assembly similar to the one in Figs. 2 and 3, but arranged in a different way, thereby providing an area having lower pressure, or being locally uncovered, for accommodation of a locally swollen area or the like;
Figs. 5a-5j are schematic top view illustrations of further embodiments of patch having various shapes; and
Fig. 6 is a schematic illustration of an embodiment of a multitude of different patches being used together.

### Detailed description of preferred embodiments

In the following detailed description, preferred embodiments of the present invention will be described. However, it is to be understood that features of the different embodiments are exchangeable between the embodiments and may be combined in different ways, unless anything else is specifically indicated. Even though in the following description, numerous specific details are set forth to provide a more thorough understanding of the present invention, it will be apparent to one skilled in the art that the present invention may be practiced without these specific details. In other instances, well known constructions or functions are not described in detail, so as not to obscure the present invention.

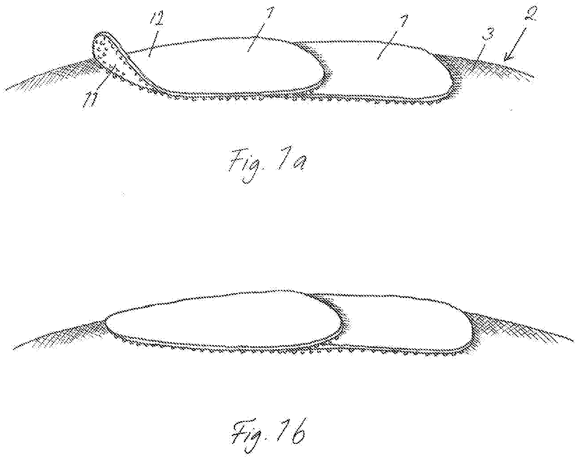

Figs. 1a and 1b show two patches 1 wherein figure 1a shows how the patch 1 is applied to a body part 2, which is wearing a garment or bandage 3.

The garment or bandage 3 may be a conventional, per se known compression device, and in particular compression garments such as compression socks, compression bandages, tubular bandages, pantyhoses, girdles and sleeves, arranged to encircle a part or all of the limb/body part to be treated by exerting a pressure towards the limb. The garment or bandage 3 is preferably made of an elastic, stretchable material, and preferably a knitted, crochet or woven textile material. In particular, it is preferred to use a garment or bandage made of a material in accordance with WO2015007335, by the same applicant. This material has the advantage of exerting a very well defined and predictable static pressure on the limb/body part, which remains the same even when the size of the body part varies. However, other materials and types of garments and bandages may be used as well to provide the static compression towards the limb/body part.

The patches 1 are arranged to provide an outer layer on top of the garment/bandage 3, and encircling all or part of the body limb/body part to be treated. The patches thereby form a second layer continuously encircling the body limb/part, thereby providing a dynamic pressure on the limb/body part.

Each patch 1 preferably has a first, inner side or skin side 11, which when applied is arranged facing the body part 1 and the underlying garment/bandage 2, and an outer side or second side 12, facing outwardly, away from the limb/body part.

The first side 11 of the patches is preferably provided with an adhering layer that adheres, at least weakly, to the surface of the garment/bandage 3 when the patch 1 is pressed against it. The patches 1 are placed so that they overlap other, already placed, patches. Further, the second side 12 is preferably also provided with an adherent layer, whereby the patches will adhere to each other even more strongly at the overlap areas. The attachment between the patches is consequently arranged to be stronger than the attachment between the patches and the underlying garment or bandage.

Fig. 1a is an illustration of a second patch in the process of being attached to the underlying garment/bandage and a previously arranged patch, and Fig. 1b shows the result when the patch has been applied.

The adherent layers may be obtained in various ways. In a preferred embodiment, the first side 11 is provided with hooks and the second side 12 with loops, to form a hook and loop type fabric, such as Velcro ^{™}. In another embodiment, both sides are provided with adhesive/glue, and preferably an adhesive which makes the patches detachable and re-attachable. It is also preferred that the adherent layer does not adhere to the skin of a human. In yet another embodiment, the first side is provided with hooks and the second side is provided with glue. In still another embodiment, the first side of the patch is provided with a weakly adherent glue while the second side is provided with glue that does not adhere strongly to the palm of the hand, but does form a strong bond with the glue provided on the first side.

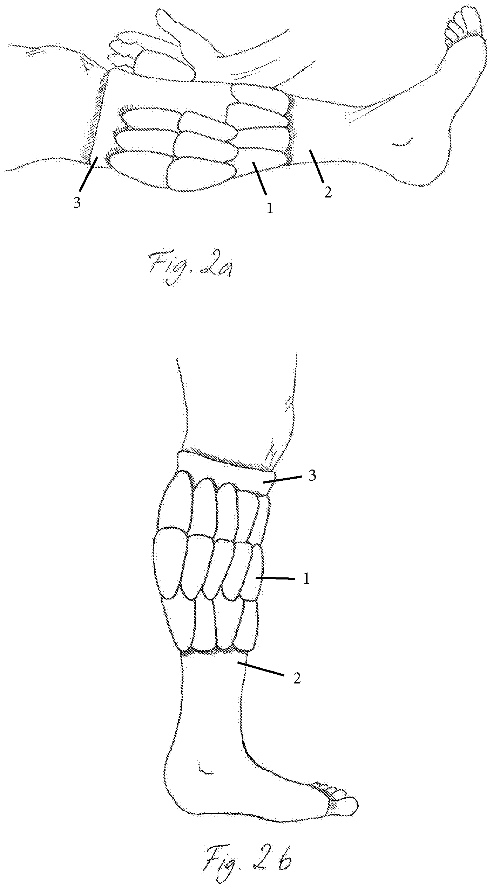

Figures 2a and 2b show the application of a preferred embodiment of the invention on a limb 2, here being a human leg. The leg is provided with a garment 3, here a tubular bandage. For application of the patches, the applicator may repeatedly place a patch 1 in the palm of one of his or her hands, with the first side facing away from the palm, and press it against the tubular bandage, in an approximately normal direction of the surface of the body part 2. This process continues until the desired section of the body part is covered by overlapping patches 1, as illustrated in Figure 2b.

In another embodiment, the garment/bandage 2 is a bandage or compression garment instead of the tubular bandage. The garment/bandage preferably provides a static pressure to the limb. However, it is also possible to use a garment/bandage which does not provide any pressure, or only a very limited pressure. Thus, in yet another embodiment, the patches are applied on a non-compression garment, such as stockings.

In order to remove the compression garment formed by the patches, it suffices to remove a handful of patches that form a longitudinal path from the proximal side to the distal side and then simply unfold the garment formed by the patches. By reversing the operation, the compression garment can be reapplied.

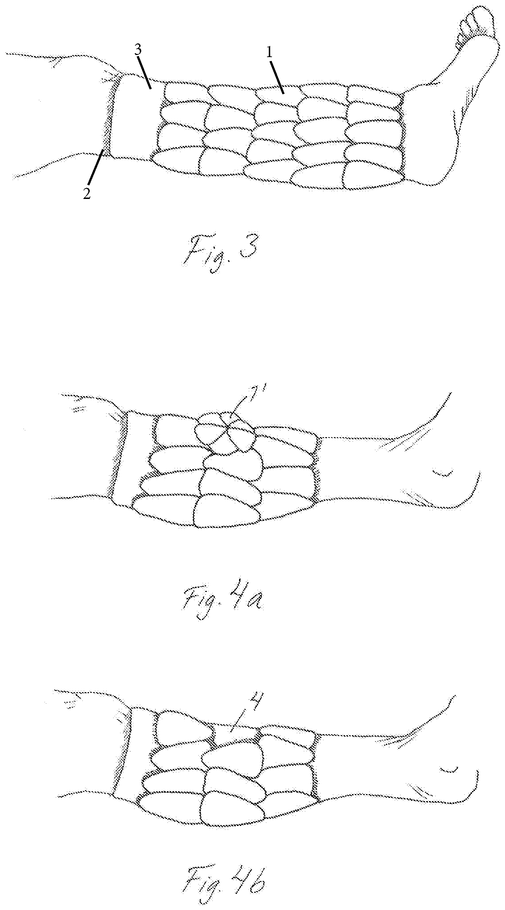

Figure 3 illustrates an embodiment of the present invention applied to a leg with a distal swelling, showing how the patches 1 may easily be arranged to adapt to the shape of the body part.

Figures 4a and 4b illustrate a situation where local considerations are applied. In Figure 4a a localized swelling is being addressed by applying patches 1' of smaller sizes over a locally swelled area, to better conform with the local curvature. When the swelling has been reduced, the smaller patches can be rearranged, or replaced with one or more larger patches, to account for the new shape of the body part.

In Figure 4b a small area 4 is left uncovered completely, for example for special clinical treatment. When treatment has been completed, the area 4 can be covered by one or more patches.

The patches 1 are preferably small in comparison with the circumferential dimension of the limb. Preferably, the patches have a maximal extension being lower than 30 cm, and preferably less than 15 cm, and most preferably less than 10 cm. Further, it is preferred that the patches are dimensioned such that at least 3 patches are required to encircle the limb/body part, and preferably at least 5, and most preferably at least 7. It is further preferred that the patches are about the same size as a human palm.

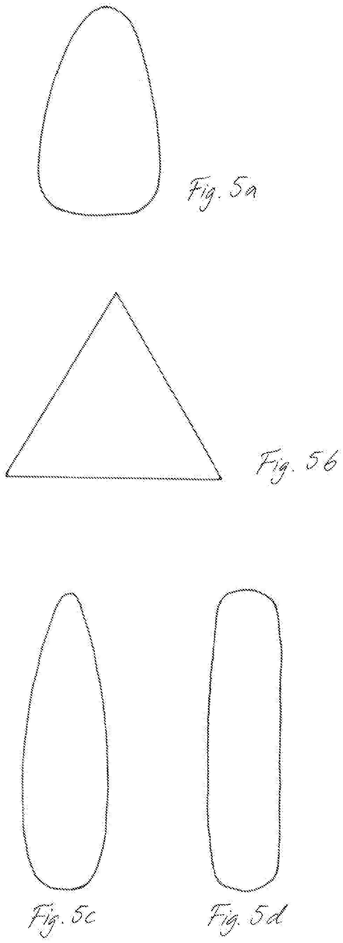

The patches may have various shapes. It is possible to use patches of only one shape to cover the limb/body part in a desired way, or to use patches having two or more different shapes. For example, one type of patches has a tapered end, and preferably having a generally triangular or drop shape. Another type of patches has a bone shape, having greater dimensions towards opposite ends, and a narrower waist there between. Some specific embodiments of the patches will be discussed in more detail in the following, with reference to Figs. 5a-j.

Figures 5a-j show examples of shapes of the individual patches in preferred embodiments.

The patch in Figure 5a is a palm sized patch that is elongated along an axis of symmetry which, upon application, will approximately align with the body parts longitudinal direction. The patch has a slightly pointed, tapered shape, which makes it easy to adapt the resulting configuration of patches to a body part with tapered shape. This generally drop shaped patch is very versatile and patches of this shape may easily be combined to cover vastly different shapes and sizes of body limbs/parts.

Figure 5b shows a patch similar to the one in Fig. 5a, but with straight edges, thereby being shaped as an equilateral triangle. Such a shape is particularly attractive from a manufacturing point of view, as it can be cut out from a larger piece of material with minimum wastage, and is still very versatile in application. In a similar embodiment, the corners of the triangle may be slightly rounded in order to eliminate sharp edges. Another embodiment may use regular n-gons or any isosceles, or even general, triangles.

Figures 5c and 5d show two patches that are more elongated than that in Figure 5a. Such shapes are particularly useful when applying the compression assembly to a long and straight body part, as it reduces the number of patches that need to be applied. To reduce the risk of the patches being stretched and applied transversally, the patches material of the patches could be such that the patches can be bent slightly. This allows for the patches to adapt to the large curvature (almost flat) in the longitudinal direction of the body part, but preferably not bend enough to adapt to the small curvature (highly curved) in the transversal direction.

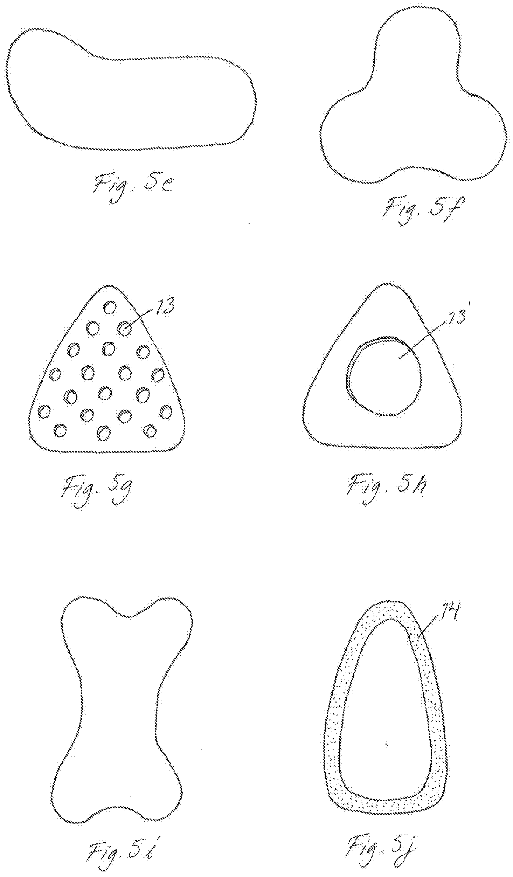

Figure 5e shows yet another embodiment of a patch, and in this embodiment the patch is more asymmetric and could be used to create more variable patterns and is particularly useful when a local clinical focus is desirable, such as in Figures 4a and 4b.

Figure 5f depicts a non-convex patch that is not elongated. Such a shape is particularly useful for body parts that have no clearly defined longitudinal direction, such as a human head.

The patches in Figures 5g and 5h are provided with holes 13, 13', which expose the underlying surface. In the embodiment of Fig. 5g, a plurality of small holes 13 are provided. A plurality of small holes does not significantly affect the compression, but does facilitate the transport of liquid, air or vapour through the patches. In the embodiment of Fig. 5h, a large hole is instead provided. Such a large opening is useful to avoid covering or applying compression over a certain area, such as a wound or the eyes or mouth, or for exposing the underlying surface in order e.g. to facilitate intravenous therapy.

A compression assembly can contain a mixture of patches, and e.g. patches with and without holes. Openings similar to the ones provided by the patches in figures 5g and 5h can also be created in situ by combining a plurality of smaller patches without holes so that openings are formed between them. However, a benefit of having the specific patches in Figures 5g and 5h is that they make it easy to explicitly determine the position of the holes.

Many other type of shapes are also feasible, and as yet another example, a patch having a bone shape is illustrated in Fig. 5i, where the dimensions towards opposite ends are greater than in the central part, thereby forming a narrower waist between the enlarged ends.

Figure 5j depicts an embodiment of a patch where a section 14 of the patch is visually or tactilely discernible. This marked section 14 is preferably arranged around the edge(s) of the patch, and preferably extends inwardly with a certain width, preferably in the range 2-15 mm, and most preferably 4-10 mm. Thus, a discernible band is formed around the perimeter of the patch. Such a discernible section along the border of the patch makes it is easy, particularly so for patches of smooth convex shape, to find an outermost patch that can easily be removed without affecting any other patch, by clearly visualize how the patches are laid, and which patches lies on top of other patches. Another use of such a discernible section is to make the user aware of a desired minimum overlap of the patches, in order to ensure a secure adhesion between the patches. Conversely, the discernible section could also be used in order to show that the overlap is excessive. To this end, it is also feasible to use more than one type of marking, thereby providing different and separately discernible sections. For example, a first section may be arranged immediately following the edge, and a second section may be arranged concentrically within the first section. Having more than one discernible section makes it particularly easy to signal both insufficient and excessive overlap. Applying a single discernible section to a patch can also automatically create multiple discernible sections, such as the three sections lying outside, on or inside an applied discernible section. Thus, a single marked section may e.g. be arranged around the edge of the patch, but at a certain distance from the edge itself.

A patch that is visually or tactilely discernible from the rest can be used to mark a certain position on the body part. Yet another use concerns the case when an individual patch is provided with coating containing a medicament. In these cases, the entire patch can be provided with visually or tactilely discernible surface, such as having a different color from the rest of the patches.

The markings may be provided in various forms, e.g. by using a different color in these sections, a different surface roughness, a different type of pattern, etc.

Markings of this type may easily be combined with any of the above-discussed various possible shapes of the patches, and also with any other desired shape.

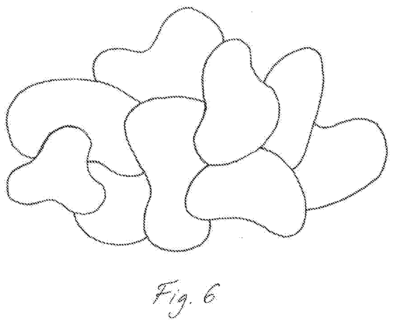

As already discussed, many of the above-discussed patches have a shape making the patches very versatile, so that the same type of patch can be used to cover many different sizes and shapes of various body limbs/parts. However, it is also possible to use a combination of patches having various shapes. Figure 6 shows, as an example, an embodiment of the present invention where patches of varying shapes and sizes are combined.

The patches can for example be produced by punching or cutting a material sheet, provided with hooks on one side and the loops on the other.

The material sheet can e.g. be a woven, knitted or non-woven fabric, or for example injection molded thermoplastic material. Further, the material can have various elastic properties, including stretching, elasticity and bending flexibility. Even more general, the material can be non-isotropic having different elastic properties in different directions, both with respect to stretching and bending. Such a non-isotropy can be aligned with the possible asymmetric shape of the patches, creating various elastic properties of the ensemble of patches as a whole. However, preferably the patches are non-elastic and non-stretchable, or elastic and stretchable only to a limited degree.

Furthermore, the shape, density, length and elastic properties of the hooks and loops will, besides the stretching elastic properties of the material in the patch, affect the resulting elastic property of the combined assembly of applied patches. If another method of adhesion than hooks and loops is used, one would obtain yet another combined elastic property.

For the patches to adhere or attach to the underlying material, the surface of that underlying material and the shape and size of the hooks on the patches is also of some relevance. The underlying material, i.e. the material of the garment or bandage, is preferably a woven or knitted fabric, forming corresponding loops, thereby forming a textured surface to which the hooks attach. The hooks can e.g. be made of loops in synthetic monofilament yarn that are cut open to create hooks, or thermoplastic injected moulded hooks in various shapes, where for example the mushroom shape tends to create less damage to the underlying hook material.

The invention has now been described with reference to specific embodiments. However, several variations of the communication system are feasible. For example, the patches may take any suitable size and shape, and many alternatives are feasible in addition to the ones discussed in the foregoing. Further, the underlying garment/bandage may be of many different types. Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements.

## Claims

1. A kit for making a limb or other body part encircling therapeutic device, for applying dynamic and/or static pressure to said limb/body part (2), comprising:
a garment or bandage (3) encircling the limb/body part (2), and preferably applying a compression to said limb/body part (2); and
a plurality of patches (1; 1') being releasably attachable to each other and to said garment or bandage (3), wherein said patches (1; 1') are releasably attachable to each other at overlap areas when arranged at least partly overlapping each other, and wherein the patches (1; 1') are dimensioned such that at least 3 patches are required to encircle the limb/body part.

2. The kit of claim 1, wherein all patches (1; 1') have limited dimensions in all directions, compared to the circumferential dimension of said limb/body part (2), and wherein each patch (1; 1') preferably has a maximal extension being lower than 30 cm, and more preferably less than 15 cm, and most preferably less than 10 cm.

3. The kit of any one of the preceding claims, wherein the patches (1; 1') are dimensioned such that at least 5 patches are required to encircle the limb/body part, and preferably at least 7.

4. The kit of any one of the preceding claims, wherein at least some of the patches have a tapered end, and preferably having a generally triangular or drop shape.

5. The kit of any one of the preceding claims, wherein the patches (1; 1') are arranged to attach more strongly to each other, at the overlap areas, and less strongly to the garment or bandage (3).

6. The kit of any one of the preceding claims, wherein the patches (1; 1') comprise at least one of adhesive and a hook and loop fabric for attachment to other patches and/or to the garment or bandage (3).

7. The kit of any one of the preceding claims, wherein the patches (1; 1') are made of a substantially inelastic, non-stretchable material or low stretchable material.

8. The kit of any one of the preceding claims, wherein the patches comprises a marking around the edges indicating a designated overlap area.

9. The kit of any one of the preceding claims, wherein at least one of the patches is provided with one or more opening(s) (13; 13').

10. The kit of any one of the preceding claims, wherein the garment or bandage (3) is made of a material having the elastic property that when the same length of unstretched elastic material is stretched to encircle circular objects with different circumferences at different yield rates the pressure exerted by the elastic material varies less than 30%, and preferably less than 20% and most preferably less than 10%, over a range of approximately circular circumferences providing a range of yield rates from λ₁ to λ₂, wherein λ₂/λ₁ > 1.8, and preferably λ₂/λ₁ > 1.9, and most preferably λ₂/λ₁ > 2.0.

11. A therapeutic pressure assembly for encircling a limb or other body part and for applying dynamic and/or static pressure to said limb/body part (2), comprising:
a garment or bandage (3) arranged to encircle said limb/body part and preferably apply a compression to said limb/body part; and
a plurality of patches (1; 1') being detachably attached to said garment or bandage (3), wherein said patches (1; 1') are arranged partly overlapping each other, and being detachably attached to each other at all overlap areas, the patches forming a second continuous encircling of the limb/body part (2), wherein the patches (1; 1') are dimensioned such that at least 3 patches are required to encircle the limb/body part.

## Patentansprüche

1. Set zur Herstellung einer therapeutischen Vorrichtung zum Umschließen eines Gliedes oder eines anderen Körperteils, um dynamischen und/oder statischen Druck auf das Glied/den Körperteil (2) auszuüben, umfassend:
ein Kleidungsstück oder eine Bandage (3), das/die das Glied/den Körperteil (2) umschließt und vorzugsweise eine Kompression auf das Glied/den Körperteil (2) ausübt; und
mehrere Pflaster (1; 1'), die lösbar aneinander und an dem Kleidungsstück oder der Bandage (3) anbringbar sind, wobei die Pflaster (1; 1') an Überlappungsbereichen lösbar aneinander anbringbar sind, wenn sie zumindest teilweise einander überlappend angeordnet sind, und wobei die Pflaster (1; 1') so bemessen sind, dass mindestens 3 Pflaster erforderlich sind, um das Glied/den Körperteil zu umschließen.

2. Set nach Anspruch 1, wobei alle Pflaster (1; 1') in allen Richtungen begrenzte Abmessungen aufweisen, verglichen mit der Umfangsabmessung des Gliedes/Körperteils (2), und wobei jedes Pflaster (1; 1') vorzugsweise eine maximale Ausdehnung von weniger als 30 cm, und noch bevorzugter von weniger als 15 cm, und am meisten bevorzugt von weniger als 10 cm aufweist.

3. Set nach einem der vorhergehenden Ansprüche, wobei die Pflaster (1; 1') so bemessen sind, dass mindestens 5 Pflaster erforderlich sind, um das Glied/den Körperteil zu umschließen, und vorzugsweise mindestens 7.

4. Set nach einem der vorhergehenden Ansprüche, wobei zumindest einige der Pflaster ein verjüngtes Ende aufweisen und vorzugsweise eine allgemein dreieckige oder tropfenförmige Form aufweisen.

5. Set nach einem der vorhergehenden Ansprüche, wobei die Pflaster (1; 1') so angeordnet sind, dass sie an den Überlappungsbereichen stärker aneinander und weniger stark an dem Kleidungsstück oder der Bandage (3) anhaften.

6. Set nach einem der vorhergehenden Ansprüche, wobei die Pflaster (1; 1') mindestens einen Klebstoff oder ein Klettgewebe zur Anbringung an anderen Pflastern und/oder an dem Kleidungsstück oder der Bandage (3) aufweisen.

7. Set nach einem der vorhergehenden Ansprüche, wobei die Pflaster (1; 1') aus einem im Wesentlichen unelastischen, nicht dehnbaren Material oder einem wenig dehnbaren Material hergestellt sind.

8. Set nach einem der vorhergehenden Ansprüche, wobei die Pflaster eine Markierung an den Rändern aufweisen, die einen bestimmten Überlappungsbereich anzeigt.

9. Set nach einem der vorhergehenden Ansprüche, wobei mindestens eines der Pflaster mit einer oder mehreren Öffnung(en) (13; 13') versehen ist.

10. Set nach einem der vorhergehenden Ansprüche, wobei das Kleidungsstück oder die Bandage (3) aus einem Material hergestellt ist, das die elastische Eigenschaft aufweist, dass, wenn dieselbe Länge des ungedehnten elastischen Materials gedehnt wird, um kreisförmige Objekte mit unterschiedlichen Umfängen bei unterschiedlichen Dehnungsraten zu umschließen, der durch das elastische Material ausgeübte Druck weniger als 30 % und vorzugsweise weniger als 20 % und am meisten bevorzugt weniger als 10 % über einen Bereich von annähernd kreisförmigen Umfängen variiert, wobei ein Bereich von Dehnungsraten von λ₁ bis λ₂ bereitgestellt wird, wobei λ₂/λ₁ > 1,8 und vorzugsweise λ₂/λ₁ > 1,9 und am meisten bevorzugt λ₂/λ₁ > 2,0.

11. Therapeutische Druckanordnung zum Umschließen eines Gliedes oder eines anderen Körperteils und zum Ausüben eines dynamischen und/oder statischen Drucks auf das Glied/den Körperteil (2), umfassend:
ein Kleidungsstück oder eine Bandage (3), das/die angeordnet ist, um das Glied/den Körperteil zu umschließen und vorzugsweise eine Kompression auf das Glied/den Körperteil auszuüben; und
mehrere Pflaster (1; 1'), die lösbar an dem Kleidungsstück oder der Bandage (3) angebracht sind, wobei die Pflaster (1; 1') teilweise einander überlappend angeordnet sind und an Überlappungsbereichen lösbar aneinander angebracht sind, wobei die Pflaster eine zweite kontinuierliche Umschließung des Glieds/Körperteils (2) bilden, wobei die Pflaster (1; 1') so bemessen sind, dass mindestens 3 Pflaster erforderlich sind, um das Glied/den Körperteil zu umschließen.

## Revendications

1. Kit de réalisation d'un dispositif thérapeutique entourant un membre ou une autre partie du corps, pour l'application d'une pression dynamique et/ou statique sur ledit membre/ladite partie du corps (2), comprenant :
un vêtement ou bandage (3) entourant le membre/la partie du corps (2), et appliquant de préférence une compression audit membre/à ladite partie du corps (2) ; et
une pluralité de patchs (1 ; 1') fixés de façon détachable les uns aux autres et audit vêtement ou bandage (3), dans lequel lesdits patchs (1 ; 1') sont fixés de façon détachable les uns aux autres au niveau de zones de chevauchement lorsqu'ils sont disposés de manière à se chevaucher au moins partiellement entre eux, et dans lequel les patchs (1 ; 1') sont dimensionnés de telle façon qu'au moins trois patchs sont nécessaires pour entourer le membre/la partie du corps.

2. Kit selon la revendication 1, dans lequel tous les patchs (1 ; 1') présentent des dimensions limitées dans toutes les directions, en comparaison avec la dimension circonférentielle dudit membre/de ladite partie du corps (2), et dans lequel chaque patch (1 ; 1') présente de préférence une extension maximale inférieure à 30 cm, et plus préférentiellement inférieure à 15 cm, et mieux encore, inférieure à 10 cm.

3. Kit selon l'une quelconque des revendications précédentes, dans lequel les patchs (1 ; 1') sont dimensionnés de telle façon que cinq patchs sont nécessaires pour entourer le membre/la partie du corps, et de préférence au moins sept.

4. Kit selon l'une quelconque des revendications précédentes, dans lequel au moins certains des patchs présentent une extrémité effilée, et présentent de préférence une forme généralement triangulaire ou de goutte.

5. Kit selon l'une quelconque des revendications précédentes, dans lequel les patchs (1 ; 1') sont conçus pour se fixer plus fortement les uns aux autres, au niveau des zones de chevauchement, et moins fortement au vêtement ou bandage (3).

6. Kit selon l'une quelconque des revendications précédentes, dans lequel les patchs (1 ; 1') comprennent l'un au moins parmi un adhésif et un crochet et un tissu à boucles pour la fixation à d'autres patchs et/ou au vêtement ou bandage (3).

7. Kit selon l'une quelconque des revendications précédentes, dans lequel les patchs (1 ; 1') sont constitués d'un matériau substantiellement inélastique, non étirable ou d'un matériau peu étirable.

8. Kit selon l'une quelconque des revendications précédentes, dans lequel les patchs comprennent un marquage autour des bords pour indiquer une zones de chevauchement désignée.

9. Kit selon l'une quelconque des revendications précédentes, dans lequel au moins certains des patchs sont pourvus d'une ou de plusieurs ouverture(s) (13 ; 13').

10. Kit selon l'une quelconque des revendications précédentes, dans lequel le vêtement ou bandage (3) est constitué d'un matériau présentant la propriété élastique, de sorte que lorsque la même longueur de matériau élastique non étiré est étirée pour entourer des objets circulaires avec des circonférences différentes à des taux de rendements différents, la pression exercée par le matériau élastique varie de moins de 30 %, et de préférence de moins de 20 %, et mieux encore de moins de 10 %, sur une plage de circonférences approximativement circulaires fournissant une plage de taux de rendement de λ₁ à λ₂, où λ₂/λ₁ > 1,8, et de préférence λ₂/λ₁ > 1,9, et mieux encore λ₂/λ₁ > 2,0.

11. Ensemble de pression thérapeutique destiné à entourer un membre ou une autre partie du corps et à appliquer une pression dynamique et/ou statique sur ledit membre/ladite partie du corps (2), comprenant :
un vêtement ou bandage (3) conçu pour entourer ledit membre/ladite partie du corps et de préférence pour appliquer une compression audit membre/à ladite partie du corps ; et
une pluralité de patchs (1 ; 1') fixés de façon détachable audit vêtement ou bandage (3), dans lequel lesdits patchs (1 ; 1') sont disposés de manière à se chevaucher au moins partiellement entre eux, et fixés de façon détachable les uns aux autres au niveau de toutes les zones de chevauchement, les patchs formant un deuxième entourage continu du membre/de la partie du corps (2), dans lequel les patchs (1 ; 1') sont dimensionnés de telle façon qu'au moins trois patchs sont nécessaires pour entourer le membre/la partie du corps.
